# EUROPEAN PATENT APPLICATION

(11) **EP 4 721 647 A1**
(43) Date of publication of application: **08.04.2026**
(21) Application number: 24856148.2
(22) Date of filing: 03.07.2024
(51) Int. Cl.: A61B 5/00, A61B 5/022, A61B 5/026, A61B 5/0285, A61M 1/16

(54) **DIAGNOSIS ASSISTANCE IMAGE DISPLAY DEVICE, TARGET LIQUID PURIFICATION DEVICE, AND TARGET LIQUID PURIFICATION SYSTEM**

(30) Priority: 24.08.2023 JP 2023136690
(71) Applicant: Physiologas Technologies, Inc., Sagamihara-shi, Kanagawa 252-0373 (JP); The Kitasato Institute, Tokyo 108-8641 (JP)
(72) Inventor: KIKUGAWA, Yuika, Tokyo 108-8641 (JP); KOBAYASHI, Kozue, Tokyo 108-8641 (JP); KOKUBO, Kenichi, Tokyo 108-8641 (JP); KUBOTA, Masaru, Tokyo 108-8641 (JP); MIYAWAKI, Kazuyoshi, Sagamihara-shi, Kanagawa 252-0373 (JP)
(74) Representative: Schön, Christoph
(86) International application number: PCT/JP2024/024146
(87) International publication number: WO 2025/041453

(57) **Abstract**

To provide a diagnosis assistance image display device for understanding a blood pressure regulation mechanism that occurs during hemodialysis and also during purification of target liquid other than blood during the purification of the target liquid, and for appropriately managing target liquid purifying actions. A diagnosis assistance image display device B displays, on a coordinate system having a first variable indicating a parameter related to peripheral circulation or temporal fluctuation thereof on a first axis and a second variable indicating a parameter related to blood flow rate or temporal fluctuation thereof on a second axis, a plot that uses variation of a motif to represent variation in a value of a third variable indicating a blood pressure at a predetermined time or temporal fluctuation of the blood pressure over a predetermined period of time, at coordinates of a value of the first variable and a value of the second variable for the predetermined time or the predetermined period of time.

## Description

### Technical Field

The present invention relates to a diagnosis assistance image display device, a target liquid purification device and a target liquid purification system.

### Background Art

Hypotension during purification of target liquid, during hemodialysis in particular, is one of the most frequent complications of hemodialysis (Non-Patent Literatures 1 and 2). It is known that for a hemodialysis patient, hypotension of during dialysis affects the prognosis of the dialysis patient (Non-Patent Literatures 3, 4, 5, 6). Various factors are relevant to hypotension during hemodialysis (Non-Patent Literatures 1, 7, 8). A patient who suffers from hypotension during dialysis can lose resistance of blood vessels and normal response of capacity to a decrease in blood volume. It is known that such a reduction in the normal vessel responsiveness is associated with various factors, such as biological incompatibility response caused by use of dialysis membrane and dialysis liquid, induction of cytokine, increased production of nitrogen monoxide, and deficiency of vasopressin (Non-Patent Literature 9).

A hemodialysis patient can also have complicated anomaly in the blood pressure regulation mechanism due to an underlying disease. Many of hemodialysis patients suffer from hypertension, which is known to be attributable not only to increase in the blood volume caused by renal dysfunction but also to a vaso-constrictive blood pressure regulation mechanism, such as the renin-angiotensin system and the sympathetic nervous system (Non-Patent Literature 10). Furthermore, there has been an observation that in a diabetic hemodialysis patient, heart rate response to a decreased blood volume may not properly function because of autonomic disorder (Non-Patent Literature 11).

### Citation List

### Non-Patent Literature

Non-Patent Literature 1: Halle MP, Hilaire D: Intradialytic Hypotension and Associated Factors among Patients on Maintenance Hemodialysis A Single - Center Study in Cameroon. Saudi Journal of Kidney Diseases and Transplantation. 31(1), 215-223, 2020.
Non-Patent Literature 2: Andrew D: Why is Intradialytic Hypotension the Commonest Complication of Outpatient Dialysis Treatments?. Kidney International Reports. 8(3), 405-418, 2023.
Non-Patent Literature 3: Cheng JL, Chih YC: Intelligent system to predict intradialytic hypotension in chronic hemodialysis. Journal of the Formosan Medical Association. 117(10), 888-893, 2018.
Non-Patent Literature 4: Johanna K, Loes MV: The prevalence of Intradialytic Hypotension in Patients on Conventional Hemodialysis: A Systematic Review with Meta-Analysis. American Journal of Nephrology. 49(6), 497-506, 2019.
Non-Patent Literature 5: Jongha P, Connie MR: A comparative effectiveness research study of the change in blood pressure during hemodialysis treatment and survival. Kidney International. 84(4), 795-802, 2013.
Non-Patent Literature 6: Shoji T, Yoshiharu T: Dialysis Hypotension and Mortality Multicenter Prospective Study. The Japanese Society for Dialysis Therapy. 36(6), 1181-1182, 2003.
Non-Patent Literature 7: Hanjie Z, Priscila P: Association of all - cause mortality with pre - dialysis systolic blood pressure and its peridialytic change in chronic hemodialysis patients. Nephrology Dialysis Transplantation. 35(9), 1602-1608, 2020.
Non-Patent Literature 8: Magdalene MA, Jennifer EF: Intradialytic Blood Pressure Abnormalities: The Highs, The Lows and All That Lies Between. American Journal of Nephrology. 42(5), 337-350, 2016.
Non-Patent Literature 9: Jeroen K, Ali B: EBPG guideline on hemodynamic instability. Nephrology Dialysis Transplantation. 22(2), ii22-ii44, 2007.
Non-Patent Literature 10: C Venkata SR, Andrew ZF: Management of hypertension in hemodialysis patients. Current Hypertension Reports. 292-298, 2009.
Non-Patent Literature 11: Alice D, Mauro FFM: Cardiovascular autonomic neuropathy in diabetes: Pathophysiology, clinical assessment and implications. World Journal of Diabetes. 12(6), 855-867, 2021.
Non-Patent Literature 12: James SS, Terrence S: Physiology, Arterial Pressure Regulation -StatPearls - NCBI Bookshelf.
Non-Patent Literature 13: Susanna C, Antonio L: Immune System Dysfunction and Inflammation in Hemodialysis Patients: Two Sides of the Same Coin. Journal of Clinical Medicine. 11(13), 3759, 2022.
Non-Patent Literature 14: Maria M, Eleftherios D: Effects of a Combined Intradialytic Exercise Training Program and Music on Cardiac Autonomic Nervous System Activity in Hemodialysis Patients. Life. 12(8), 1276, 2022.
Non-Patent Literature 15: Peter K: Cause and Consequences of Sympathetic Hyperactivity in Chronic Kidney Disease. Blood purification. 24(1), 95-99, 2005.
Non-Patent Literature 16: Aaron I, Raelene E: Diabetic Autonomic Neuropathy. Diabetes Care. 26(5), 1553-1579, 2003.

### Summary of Invention

### Technical Problem

In addition to multiple factors relevant to blood pressure fluctuations during hemodialysis, the blood pressure regulation mechanisms of hemodialysis patients also vary depending on their underlying diseases. Accordingly, for proper management of various types of hypotension during hemodialysis, it is necessary to know the individual blood pressure regulation mechanisms of dialysis patients.

An object of the present invention is therefore to provide a diagnosis assistance image display device, a target liquid purification device and a target liquid purification system for understanding a blood pressure regulation mechanism that occurs during hemodialysis and also during purification of target liquid other than blood during the purification of the target liquid, and for appropriately managing target liquid purifying actions.

### Solution to Problem

To solve the problem above, a diagnosis assistance image display device according to the present invention is characterized by having the following limitations of the invention.

The diagnosis assistance image display device according to the present invention displays, on a coordinate system having a first variable indicating a parameter related to peripheral circulation or temporal fluctuation thereof on a first axis and a second variable indicating a parameter related to blood flow rate or temporal fluctuation thereof on a second axis, a plot that uses variation of a motif to represent variation in a value of a third variable indicating a blood pressure at a predetermined time or temporal fluctuation of the blood pressure over a predetermined period of time, at coordinates of a value of the first variable and a value of the second variable for the predetermined time or the predetermined period of time.

In the diagnosis assistance image display device configured as described above, the motif preferably represents time-series fluctuations of at least one of the first variable, the second variable, and the third variable.

In either of the diagnosis assistance image display devices described above, a region identifying a factor that affects blood pressure is preferably displayed on the coordinate system, the region enclosed by a line.

To solve the problem above, a target liquid purification device of the present invention is characterized by having the following limitations of the invention.

The target liquid purification device of the present invention is a target liquid purification device for removing at least one target substance from unpurified target liquid of a patient, the target liquid purification device including:
a first measurement device for measuring a first variable indicating a parameter related to peripheral circulation of the patient or temporal fluctuation thereof;
a second measurement device for measuring a second variable indicating a parameter related to blood flow rate of the patient or temporal fluctuation thereof; and
a third measurement device for measuring a third variable indicating a parameter related to blood pressure of the patient or temporal fluctuation thereof.

In the target liquid purification device configured as described above, the target liquid purification device includes a control unit that controls operations of the target liquid purification device based on at least one variable among the first variable measured by the first measurement device, the second variable measured by the second measurement device, and the third variable measured by the third measurement device.

To solve the problem above, a target liquid purification system of the present invention is characterized by having the following limitations of the invention.

The target liquid purification system of the present invention includes:
the diagnosis assistance image display device described in any of the aspects above; and
the target liquid purification device described in any of the aspects above.

In the target liquid purification system configured as described above, the target liquid purification system preferably performs processing for storing, in a storage device, at least one variable among the first variable measured by the first measurement device, the second variable measured by the second measurement device, and the third variable measured by the third measurement device.

### Advantageous Effects of Invention

Blood pressure is generally affected by peripheral circulation and blood flow rate. Peripheral circulation is known to be controlled by the sympathetic nervous system, the renin-angiotensin system, and vasopressin, and also systems other than the sympathetic nervous system are involved in it (Non-Patent Literature 12). Blood flow rate is primarily controlled by the sympathetic nervous system.

Accordingly, the diagnosis assistance image display device, the target liquid purification device and the target liquid purification system described in any of the above aspects enable learning of change in the mechanism of blood pressure fluctuation by looking at a condition related to peripheral circulation and a condition related to blood flow rate at the same time as blood pressure fluctuation. It is then possible to manage a target liquid purification process and the like according to the mechanism of blood pressure fluctuation, which in turn enables a target liquid purification process to be carried out safely while managing the blood pressure appropriately. Additionally, detrimental effects on patients that may result from medical accidents and the like can be reduced because the condition of a patient receiving target liquid purification, such as a dialysis patient, can be quickly recognized.

### Brief Description of Drawings

FIG. 1 schematically shows a target liquid purification system as an embodiment of the present invention.
FIG. 2 schematically shows a main unit of the target liquid purification system as an embodiment of the present invention.
FIG. 3 shows an example of a displayed image on a diagnosis assistance image display device according to the present invention.
FIG. 4 shows an example of a displayed image that shows regions of a range of blood pressure abnormality due to fluctuations in vasopressin, a range of blood pressure abnormality due to fluctuations of the renin-angiotensin system, a range of blood pressure abnormality due to fluctuations of the sympathetic nervous system, and a range of blood pressure abnormality due to vasodilating action on the coordinate system in FIG. 3, where the regions are enclosed by lines.
FIG. 5 shows time-series fluctuations in arterial blood pressure for an extracorporeal circulation group and a target liquid purification group in an experiment.
FIG. 6 shows time-series fluctuations in blood oxygen saturation for the extracorporeal circulation group and the target liquid purification group in the experiment.
FIG. 7 shows time-series fluctuations in heart rate for the extracorporeal circulation group and the target liquid purification group in the experiment.
FIG. 8 shows ΔBP-ΔSpO₂-ΔHR and time-series fluctuations in arterial blood pressure for the extracorporeal circulation group.
FIG. 9 shows ΔBP-ΔSpO₂-ΔHR and time-series fluctuations in arterial blood pressure for the target liquid purification group.

### Description of Embodiments

An embodiment of the present invention is now described with drawings.

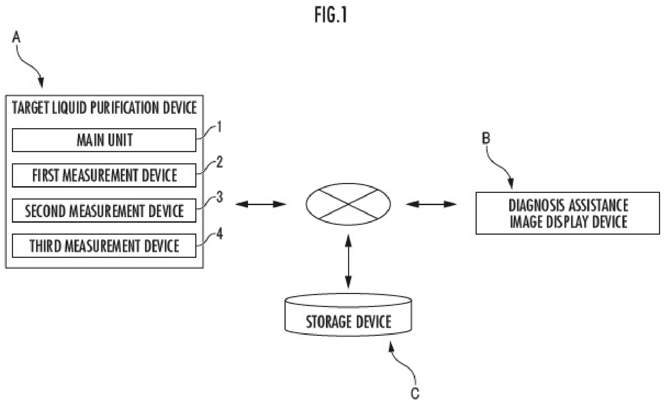

A target liquid purification system as a first embodiment of the present invention shown in FIG. 1 comprises a target liquid purification device A and a diagnosis assistance image display device B. The target liquid purification device A and the diagnosis assistance image display device B are connected with each other via their respective wireless communication devices (not shown). Any wireless communication scheme, such as Internet, Bluetooth (a registered trademark), and infrared communication, may be used as the way of wireless communication. Aside from wireless communication, the target liquid purification device A and the diagnosis assistance image display device B may also be connected over wired connection via a lead wire and the like.

### (Configuration of the target liquid purification device A)

The target liquid purification device A comprises a main unit 1 which removes at least one target substance from unpurified target liquid of a patient, a first measurement device 2 for measuring a first variable indicating a parameter related to peripheral circulation of the patient or temporal fluctuation thereof, a second measurement device 3 for measuring a second variable indicating a parameter related to the blood flow rate of the patient or temporal fluctuation thereof, and a third measurement device 4 for measuring a third variable indicating a parameter related to the blood pressure of the patient or temporal fluctuation thereof. The target liquid purification system performs processing for storing at least one variable among the first variable measured by the first measurement device 2, the second variable measured by the second measurement device 3 and the third variable measured by the third measurement device 4 in a storage device C.

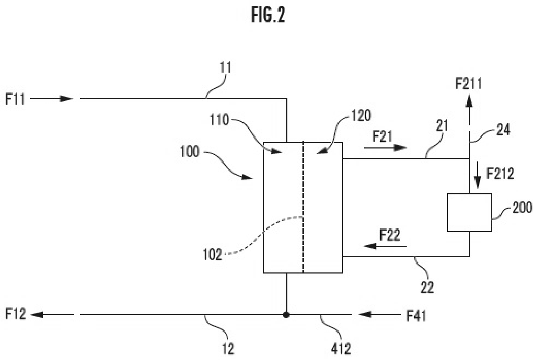

FIG. 2 shows an example of the main unit 1 of the target liquid purification device A in this embodiment. As shown in FIG. 2, the main unit 1 comprises a separator 100, an unpurified target liquid path 11, a purified target liquid path 12, a regenerating section 200, a filtration and dialysis liquid path 21, a regenerated liquid path 22, a waste liquid path 24, and a first replenishing liquid path 412. The regenerating section 200, the filtration and dialysis liquid path 21, the regenerated liquid path 22, the waste liquid path 24, and the first replenishing liquid path 412 may be omitted.

The separator 100 is divided into a first section 110 and a second section 120 by a target liquid purifying membrane 102. The target liquid purifying membrane 102 has a pore size that allows passage of the target substance contained in unpurified target liquid F11 (e.g., blood, plasma, dialysis waste liquid, blood filtration liquid) therethrough. The "target substance" is a morbific substance that builds up in a person's body when suffering a disease, e.g., at least one ion of potassium ion, ammonium ion, calcium ion, magnesium ion, phosphate ion, hydrogen carbonate ion and organic acid ion, urea, creatinine, uric acid, peptide, and protein. The separator 100 is configured to pass unpurified target liquid F11 containing the target substance through the target liquid purifying membrane 102 from the first section 110 and introduce it into the second section 120, thus producing filtration and dialysis liquid F21, and to remove the target substance contained in the unpurified target liquid F11 through such passage, thus producing purified target liquid F12.

The unpurified target liquid path 11 is configured to introduce the unpurified target liquid F11 drawn from the patient's body into the first section 110 of the separator 100. The purified target liquid path 12 is configured to draw the purified target liquid F12 from the first section 110 of the separator 100 and introduce it into the patient's body. The separator 100 consists of a dialyzer, for example. The first section 110 is an internal space of hollow fiber made from dialysis membrane, and the second section 120 is a space around the hollow fiber in which dialysis liquid and/or regenerated liquid flows.

In this embodiment, the separator 100 is configured as a "counter flow separator", where a flowing direction of the unpurified target liquid F1 in the first section 110 and a flowing direction of the regenerated liquid F22 in the second section 120 are opposite or reverse to each other. However, in another embodiment, the separator 100 may also be configured as a "parallel flow separator", where the flowing direction of the unpurified target liquid F11 in the first section 110 and the flowing direction of the regenerated liquid F22 in the second section 120 are parallel or the same.

The regenerating section 200 may include adsorbent for adsorbing the target substance. The adsorbent may be active carbon or adsorbent based on active carbon as base material, porous adsorbent, cation exchange resin, anion exchange resin, zirconia-based ceramic, adsorbent made of zeolite, and a mixture thereof. The regenerating section 200 may be configured to remove at least part of the target substance by bringing part of filtration and dialysis liquid (a second liquid F212) into contact with the adsorbent and produce regenerated liquid F22 with a reduced concentration of the target substance.

The filtration and dialysis liquid path 21 is configured to draw the filtration and dialysis liquid F21 from the second section 120 of the separator 100 and introduce the second liquid F212, which is part of the liquid, to the regenerating section 200. The waste liquid path 24 branches from the filtration and dialysis liquid path 21 and is configured to discard the first liquid F211 as waste liquid out of the first liquid F211 and the second liquid F212 which result from the separation of the filtration and dialysis liquid F21. The flow rate of waste liquid on the waste liquid path 24 (the amount of discarded filtration and dialysis liquid F21) is also based on the amount of inflow of replenishing liquid (fresh dialysis liquid) into the regenerated liquid path 22 and/or the amount of outflow of the regenerated liquid F22 from the regenerated liquid path, and may be controlled appropriately in view of the flow rate of the regenerated liquid F22 in the second section 120 of the separator 100. The regenerated liquid path 22 is configured to introduce the regenerated liquid F22 from the regenerating section 200 into the second section 120 of the separator 100.

The first replenishing liquid path 412 is configured to directly introduce the first replenishing liquid F41 into the purified target liquid path 12. "Replenishing liquid" means fresh dialysis liquid or replenishing liquid for filtration, for example. The first replenishing liquid F41 may be introduced from the purified target liquid path 12 located downstream of the separator 100, or introduced from the unpurified target liquid path 11 located upstream of the separator 100, or the first replenishing liquid F41 may also be omitted.

The main unit may also be in a single-path form, omitting the regenerating section 200, the filtration and dialysis liquid path 21, the regenerated liquid path 22, the waste liquid path 24, and the first replenishing liquid path 412. As mentioned earlier, the regenerating section 200, the filtration and dialysis liquid path 21, the regenerated liquid path 22, the waste liquid path 24, and the first replenishing liquid path 412 are not essential components of the present invention and may be omitted.

The first measurement device 2 is a device for measuring the first variable indicating a parameter related to the peripheral circulation of the patient or temporal fluctuation thereof. The parameter related to the peripheral circulation of the patient can be blood oxygen saturation (SpO₂), an indicator measured by a laser Doppler blood flow meter or an ultrasonic Doppler blood flow meter, or a perfusion indicator, for example. In this embodiment, the parameter related to the peripheral circulation of the patient is preferably blood oxygen saturation for the sake of ease of measurement. Preferably, the first measurement device 2 is attached to the patient's hand and measures the blood oxygen saturation. The first measurement device 2 is preferably connected with the main unit 1 of the target liquid purification device A over a lead wire, and the parameter related to the peripheral circulation of the patient is stored in the storage device C over the lead wire.

Although the first measurement device 2 is attached to the patient's hand in the embodiment above, it may also be provided in a portion of a blood circuit of the target liquid purification device or may be attached to a body part of the patient other than the hand. Also, although the first measurement device 2 is connected to the main body of the target liquid purification device A over a lead wire in the embodiment above, it may be connected to the main unit 1 of the target liquid purification device A in any wireless communication scheme, such as Internet, Bluetooth (a registered trademark), and infrared communication.

The second measurement device 3 is a device for measuring the second variable indicating a parameter related to the blood flow rate of the patient or temporal fluctuation thereof. Here, the parameter related to the blood flow rate of the patient can be heart rate (HR) or cardiac output, for example. For the present invention, the parameter related to the blood flow rate of the patient is preferably the heart rate for the sake of ease of measurement. The second measurement device 3 can also perform measurement simultaneously from a blood oxygen saturation meter attached to the patient's hand. The second measurement device 3 is preferably connected with the main unit 1 of the target liquid purification device A over a lead wire, and the parameter related to the blood flow rate of the patient is stored in the storage device C over the lead wire.

Although the second measurement device 3 is attached to the patient's hand in the embodiment above, it may also be provided in a portion of a blood circuit of the target liquid purification device or may be attached to a body part of the patient other than the hand. Also, although the second measurement device 3 is connected to the main unit 1 of the target liquid purification device A over a lead wire in the embodiment above, it may be connected to the main unit 1 of the target liquid purification device A in any wireless communication scheme, such as Internet, Bluetooth (a registered trademark), and infrared communication.

The third measurement device 4 is a device for measuring the third variable indicating a parameter related to the blood pressure of the patient or temporal fluctuation thereof. Here, the parameter related to the blood pressure of the patient can be the blood pressure in an upper arm, for example. In the invention, the third measurement device 4 preferably measures the blood pressure in the upper arm of the patient in particular, and is preferably placed near the patient's upper arm. The third measurement device 4 is preferably connected with the main unit 1 of the target liquid purification device A over a lead wire, and the parameter related to the blood pressure of the patient is stored in the storage device C over the lead wire.

Although the third measurement device 4 is placed near the upper arm in the embodiment above, it may also be provided in a portion of the blood circuit of the target liquid purification device or be provided in a portion where it can measure a site where an artery or a vein is located other than the upper arm. Also, although the third measurement device 4 is connected to the main unit 1 of the target liquid purification device A over a lead wire in the embodiment above, it may be connected to the main unit 1 of the target liquid purification device A in any wireless communication scheme, such as Internet, Bluetooth (a registered trademark), and infrared communication.

Preferably, each of the first measurement device 2, the second measurement device 3 and the third measurement device 4 performs sampling at the intervals of 10 seconds to 60 minutes from the viewpoint of accurately monitoring the patient's condition. More preferably, they perform sampling at the intervals of 1 to 20 minutes from the viewpoint of data storage and processing.

The target liquid purification device A may further include a control unit for controlling a target liquid purification process. The control unit includes a storage device C (e.g., memory such as RAM, ROM, EEPROM, an SSD, an HDD) to store and hold programs (software) and data, a computational processing unit (e.g., a single-core processor, a multi-core processor, a CPU) that reads a necessary program and/or data from the storage device C and performs certain computational processing, and an I/O circuit, for example. The control unit controls the flow rate or the amount of removal of replenishing liquid in a target liquid purification process and controls the operation of the entire target liquid purification device, as will be discussed later. Some of the storage device C, the computational processing unit, the I/O circuit and other components may be provided independently of the control unit or may be configured integrally with it. The control unit includes an anomaly detection unit. The anomaly detection unit detects any anomaly during target liquid purification (e.g., an abrupt blood pressure fluctuation, low blood pressure condition) based on the first variable, the second variable, or the third variable, and reports the anomaly.

### (Configuration of the diagnosis assistance image display device)

The diagnosis assistance image display device B can be a personal computer, a mobile phone (smartphone) or the like, including any kind of information terminal device. The diagnosis assistance image display device B displays different kinds of information on a target liquid purification process, such as the flow rate of replenishing liquid, time elapsed in target liquid purification, the total amount of inflow of the replenishing liquid, composition of the unpurified target liquid F11, and composition of the purified target liquid F12 in a target liquid purification process. The diagnosis assistance image display device B may also be provided in a remote device that remotely operates or manages the target liquid purification device A.

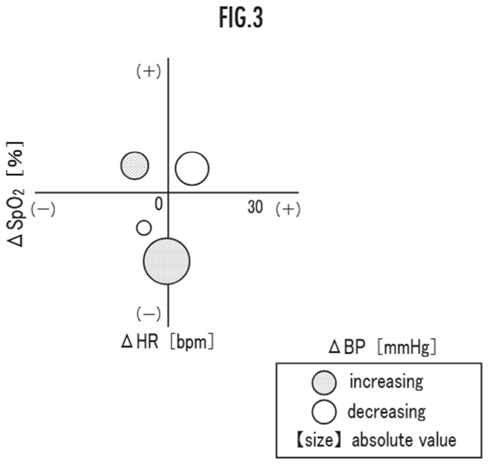

The diagnosis assistance image display device B displays, on a coordinate system having a first variable indicating a parameter related to peripheral circulation or temporal fluctuation thereof on a first axis and a second variable indicating a parameter related to blood flow rate or temporal fluctuation thereof on a second axis, a plot that uses variation of a motif to represent variation in a value of a third variable indicating a blood pressure at a predetermined time or temporal fluctuation of the blood pressure over a predetermined period of time, at coordinates of a value of the first variable and a value of the second variable for the predetermined time or the predetermined period of time. FIG. 3 show an example of an image displayed on the diagnosis assistance image display device B. The coordinate system shown in FIG. 3 has temporal difference (ΔHR) in the heart rate as the second variable over a predetermined period of time on the vertical axis, and temporal difference (ΔSpO₂) in the blood oxygen saturation as the first variable over the predetermined period of time on the horizontal axis. While in this example the vertical axis is ΔHR and the horizontal axis is ΔSpO₂, the variables indicated by the vertical axis and the horizontal axis may be interchanged. In addition, although the blood oxygen saturation and the heart rate are on the horizontal axis and the vertical axis, respectively, the first variable indicating a parameter related to peripheral circulation other than the blood oxygen saturation or temporal fluctuation thereof may be on the horizontal axis, and the second variable indicating a parameter related to blood flow rate other than the heart rate or temporal fluctuation thereof may be on the vertical axis, as mentioned earlier.

FIG. 3 further includes plotting of motifs that represent the variation in the value of the temporal difference (ΔBP) in the blood pressure as the third variable over a predetermined period of time at the coordinates of the value of the first variable and the value of the second variable for a predetermined time or a predetermined period of time. Here, the motifs are represented as circles, where the radius of a circle increases as the absolute value of the temporal difference in the blood pressure increases, and the variation in the third variable is represented such that the color of a circle is indicated in gray when the temporal difference in the blood pressure is positive and in white when it is negative.

That is to say, the diagnosis assistance image display device B displays the temporal fluctuation of the patient's heart rate and the temporal fluctuation of the blood oxygen saturation during target liquid purification or before or after target liquid purification by showing motifs on the coordinate system in the form of a scatter diagram. The motifs shown in FIG. 3 are indicated in the form of a bubble chart, where a motif is rendered in a colored form when plotted if the temporal fluctuation (ΔBP) of the blood pressure is positive and in white if it is negative, with the size of plotting representing the absolute value of the temporal difference in the blood pressure over a predetermined period of time.

Although the first variable, the second variable, and the third variable in this embodiment are represented as temporal differences for a predetermined period of time, they may also be time derivative, N-th time derivative (N being an arbitrary integer, including 0), discretized N-th time derivative for a predetermined period of time. The difference in this embodiment is preferably forward difference, but it may be any kind of difference, such as backward difference and center difference.

The diagnosis assistance image display device B is preferably represented in the form of a bubble chart, but it may display any type of diagram, such as a heatmap, a radar chart, a bar graph, and a line graph.

Such a motif as mentioned above may be made up of multiple motif portions. For example, after displaying a motif for one predetermined period of time as one motif portion on the diagnosis assistance image display device B, a motif for the next predetermined period of time can be displayed as another motif portion simultaneously with the one motif portion or as a moving image. In doing so, if one motif portion and another motif portion are displayed simultaneously, the one motif portion and the other motif portion may be represented so as to indicate the time-series fluctuations of at least one of the first variable, the second variable and the third variable. For example, the one motif portion and the other motif portion may be connected by an arrow line, or an effect such as changing their colors with time may be applied. There may be two or two or more motif portions. When one motif portion and another motif portion are displayed as moving images, that is, when the motif is displayed as dynamic motifs, the motif may be produced as animation, such as after the one motif portion being displayed, the other motif portion being displayed fractions of a second later.

The diagnosis assistance image display device B may also display a region identifying a factor that affects blood pressure on the coordinate system described above, the region enclosed by a line. Factors that affect blood pressure include a biological hormone of the vasopressin system, a biological hormone of the renin-angiotensin system, fluctuations of the sympathetic nervous system, and vasodilating action.

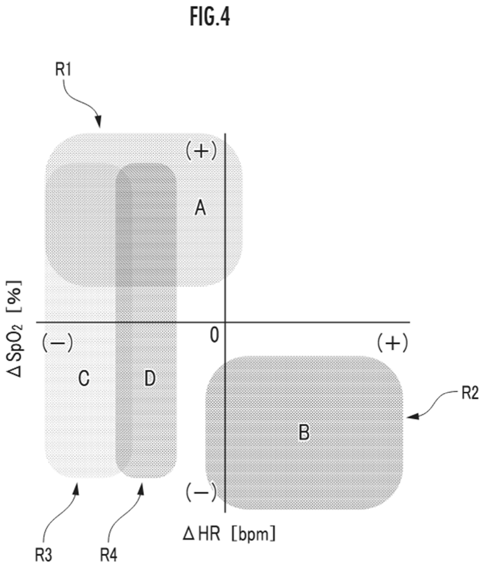

FIG. 4 shows an embodiment of a displayed image that shows, on the coordinate system, a region in which a blood pressure fluctuation due to fluctuations in the biological hormone of the vasopressin system is determined to have occurred, a region in which a blood pressure fluctuation due to fluctuations in the biological hormone of the renin-angiotensin system is determined to have occurred, a region in which a blood pressure fluctuation due to fluctuation of the sympathetic nervous system is determined to have occurred, and a region in which a blood pressure fluctuation due to vasodilating action is determined to have occurred, each region being enclosed by a line. Here, region R1 is a range of blood pressure abnormality due to fluctuation of the sympathetic nervous system. In general, when the sympathetic nerves are excited, the heart rate tends to increase and peripheral blood vessels tend to dilate (decrease in the blood oxygen saturation). Region R2 is a range of blood pressure abnormality due to fluctuation of the sympathetic nervous system. In general, when the sympathetic nerves are suppressed, the heart rate tends to increase and peripheral blood vessels tend to constrict (increase in the blood oxygen saturation). Region R3 is a range of blood pressure abnormality due to activation of the renin-angiotensin system. In general, when the renin-angiotensin system are activated, peripheral blood vessels constrict (the blood oxygen saturation increases), whereas the heart rate does not vary. Consequently, ΔHR changes from a positive value to a negative value. Hence, in the region R3, ΔHR changes from a positive value to a negative value and ΔSpO₂ assumes a negative value. Region R4 is a range of blood pressure abnormality due to vasopressin. In general, the heart rate is not affected by vasopressin. Hence, in the region R4, ΔHR changes from a positive value to a negative value and ΔSpO₂ assumes a negative value. Since the effect of vasoconstriction caused by vasopressin on ΔSpO₂ is essentially weaker than the effect of the renin-angiotensin system, the region R4 takes a value close to the origin compared to the region R3.

In addition to the foregoing aspects, the diagnosis assistance image display device B may further add a region of a normal range or a region of blood pressure abnormality that is caused by the effect of other biological hormone on the coordinate system. The diagnosis assistance image display device B may further display a graph related to the time-series fluctuations of the first variable, the time-series fluctuations of the second variable, or the time-series fluctuations of the third variable.

The diagnosis assistance image display device B may also display a diagram that is based on the values of the first variable, the second variable and the third variable for a target liquid purification process in the past stored in the storage device C. In other words, it may display the blood pressure fluctuations of the patient associated with past target liquid purification and factors thereof prior to target liquid purification.

### EXAMPLES

Next, examples of this embodiment are described with drawings.

### (Subjects)

Sprague Dawley rats (male, 10-week or older, CLEA Japan, Inc.) were used for experiment. The growing environment of the rats is an environment in a constant-temperature, constant-humidity room at a temperature of 25°C (a relative humidity of 50%), where light period: dark period is 12:12 hours. During an operation, the rats were anesthetized with 3% isoflurane. During perfusion, the rats were anesthetized with 1.5% isoflurane.

The experiment for this embodiment was conducted as per "Fundamental Guidelines for Proper Conduct of Animal Experiments and Related Activities in Academic Research Institutions". The present study was approved by the Institutional Animal Care and Use Committee at Kitasato University of School of Allied Health Science (Approval No. 19-05-3).

### (Experimental groups)

The experiment was conducted on an extracorporeal circulation group and a target liquid purification group as experimental groups. The extracorporeal circulation group is an experimental group using rats whose blood was collected from a femoral artery and returned to a femoral vein through the unpurified target liquid path 11 (LABORAN (a registered trademark), silicone tube 1×3, 9-869-03, AS ONE Corporation, Japan). That is to say, the extracorporeal circulation group is a group that undergoes the experiment without going through a separator. The target liquid purification group is an experimental group that undergoes extracorporeal circulation, where blood collected from the femoral artery as unpurified target liquid F11 is introduced into the separator 100 (the target liquid purifying membrane 102 is polyether sulfone, with a membrane area of 141 cm², the number of hollow fibers of 140, and an effective length of 160 mm) through the unpurified target liquid path 11 (LABORAN (a registered trademark), silicone tube 1×3, 9-869-03, AS ONE Corporation, Japan), and then the purified target liquid F12 drawn from the separator 100 is returned to the femoral vein through the purified target liquid path 12 (LABORAN (a registered trademark), silicone tube 1×3, 9-869-03, AS ONE Corporation, Japan). The target liquid purification group underwent the experiment without going through the regenerating section 200, the filtration and dialysis liquid path 21, the regenerated liquid path 22, the waste liquid path 24, and the first replenishing liquid path 412. There was no noticeable difference in the sizes of rats between the two groups (the extracorporeal circulation group: 398.0 ± 14.2 g, the target liquid purification group: 348.1 ± 25.1 g).

### (Experimental environment)

The unpurified target liquid path 11 was sealed and disinfected with peracetic acid overnight and connected with the main body of the target liquid purification device A, which was sterilized with X-ray. The unpurified target liquid path 11, the main body of the target liquid purification device A and the purified target liquid path 12 were primed with lactated Ringer solution (Lactec Injection, Otsuka Pharmaceutical Co., Ltd.). The amount of priming was 1.8 mL for the extracorporeal circulation group and 3.6 mL for the target liquid purification group.

### (Experimental outline of the experiment)

The rats of each group were anesthetized with 3% isoflurane and cannulated with a 24G intravenous canula (Surflo Flash (a registered trademark), intravenous injection cannula 24G×3/4, Terumo) in the caudal vein. Next, the inguinal region on one side was incised and the femoral vein and the femoral artery were separated. The femoral vein was cannulated with a 20G infusion cannula (Surflo Flash (a registered trademark), infusion cannula 20G×1 1/4, Terumo). The femoral artery was cannulated with a 24G infusion cannula. After finishing the operation, a 4-hour circulation experiment was conducted on each experimental group. Using a peristaltic pump (SJ-1211H, ATTO Corporation, Japan), extracorporeal circulation was performed at a blood flow rate (QB) of 1.0 mL/min. The replenishing liquid path 41 (dialysis liquid) was filled with lactated Ringer solution and closed during contact with blood so that it does not flow on the dialysis liquid side of the dialyzer. Heparin was added for preventing coagulation of blood. Heparin was injected in bolus in 0.50 unit/g at the start of circulation and continually injected one hour after the start of circulation. During circulation, activated clotting time (ACT) was measured every one hour and the amount of continual administration of heparin was adjusted so that ACT is around 350 seconds.

### (Measurement conditions)

As the third variable, the arterial blood pressure was measured from the femoral artery and its fluctuations were recorded. The pulse oximeter sensor probe of a pulse oximeter and heart rate monitor (PhysioSuite (a registered trademark) for Mice & Rats, Kent Scientific) was attached to a rat's hand, and the blood oxygen saturation (SpO₂) for determining the first variable and the heart rate (HR) for determining the second variable were measured. All entries were recorded every 15 minutes. This Example used forward temporal difference (ΔSpO₂) in the blood oxygen saturation at 15 minutes as the first variable, forward temporal difference (ΔHR) in the heart rate at 15 minutes as the second variable, and forward temporal difference (ΔBP) in the blood pressure at 15 minutes as the third variable.

### (Results)

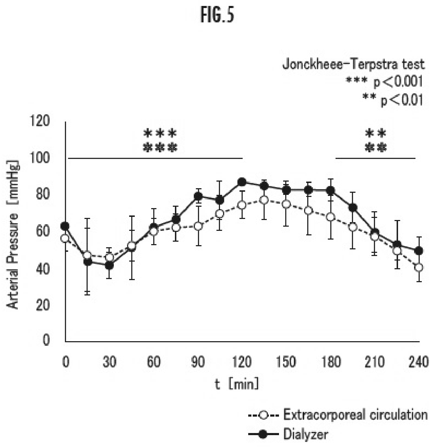

FIG. 5 shows the time-series fluctuations of the arterial blood pressure for the extracorporeal circulation group (Extracorporeal circulation) versus the target liquid purification group (Dialyzer) in the present experiment. Referring to FIG. 5, the arterial blood pressure exhibited a significant rising trend 2 hours after the start of perfusion and a significant falling trend 3 to 4 hours after the start of perfusion both for the extracorporeal circulation group and the target liquid purification group.

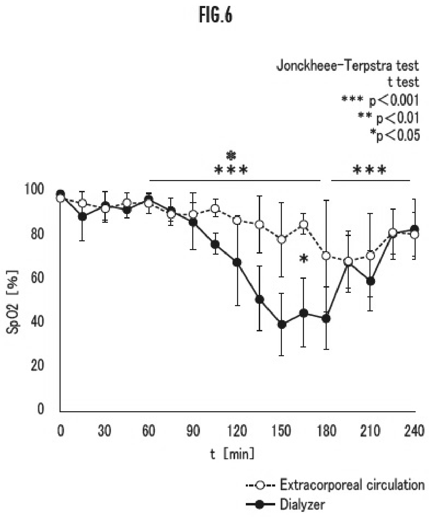

FIG. 6 shows the time-series fluctuations of the blood oxygen saturation (SpO₂) for the extracorporeal circulation group (Extracorporeal circulation) versus the target liquid purification group (Dialyzer) in this experiment. Referring to FIG. 6, the blood oxygen saturation exhibited a significant falling trend 1 to 3 hours after the start of perfusion both for the extracorporeal circulation group and the target liquid purification group. Also, at 165 minutes after the start of extracorporeal circulation, the blood oxygen saturation of the target liquid purification group was significantly lower than that of the extracorporeal circulation group.

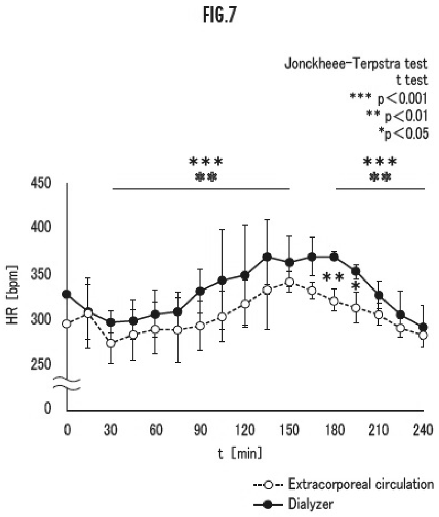

FIG. 7 shows the time-series fluctuations of the heart rate (HR) for the extracorporeal circulation group (Extracorporeal circulation) versus the target liquid purification group (Dialyzer) in this experiment. Referring to FIG. 7, the heart rate exhibited a significant rising trend from 30 minutes through 150 minutes after the start of perfusion both for the extracorporeal circulation group and the target liquid purification group, and exhibited a significant falling trend from 3 hours through to 4 hours after the start of perfusion. At three hours (180 minutes) after and 195 minutes after the start of perfusion, the heart rate of the target liquid purification group was significantly higher than that of the extracorporeal circulation group. The arterial blood pressure during a perfusion experiment exhibited similar fluctuations for the extracorporeal circulation group and the target liquid purification group.

Although there were differences in the blood oxygen saturation and the heart rate between the two groups, the overall trends of fluctuation were similar. Plotting data on ΔBP-ΔSpO₂-ΔHR correlation diagrams showed a more noticeable difference between the extracorporeal circulation group and the target liquid purification group.

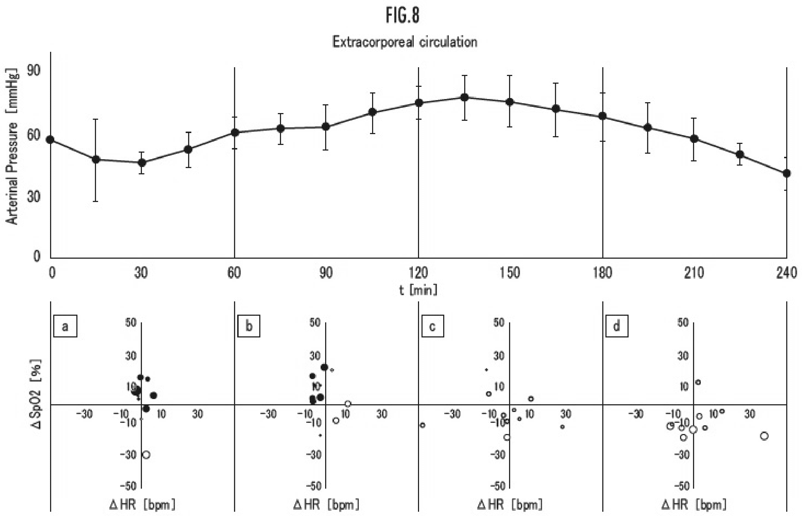

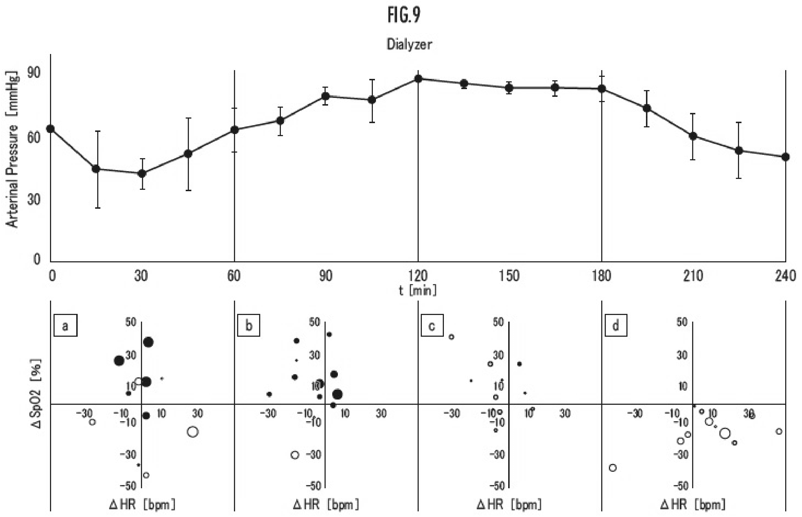

FIG. 8 shows the ΔBP-ΔSpO₂-ΔHR at 60 minutes and the time-series fluctuations of the arterial blood pressure for the extracorporeal circulation group. FIG. 9 shows the ΔBP-ΔSpO₂-ΔHR at 60 minutes and the time-series fluctuations of the arterial blood pressure for the target liquid purification group. For the ΔBP-ΔSpO₂-ΔHR described in FIGS. 8 and 9, a black circle indicates that the temporal difference in the heart rate is positive, a white circle indicates that the temporal difference in the heart rate is negative, and the size of the radius of the circle indicates the magnitude of the absolute value of the temporal difference in the heart rate. On the ΔBP-ΔSpO₂-ΔHR correlation diagram at 0 to 1 hour after the start of circulation (FIGS. 8a and 9a), an increase in the heart rate as well as an increase in the arterial blood pressure were observed for the target liquid purification group (FIG. 8a). For the target liquid purification group, the plots are located in the region R1 of FIG. 4.

On the correlation diagram at one to two hours after the start of circulation (FIGS. 8b and 9b), decrease in the blood oxygen saturation as well as increase in the heart rate were observed and the plots were located in region R1. Decrease in the blood oxygen saturation is more noticeable with the target liquid purification group (FIG. 9b), and the plots are located in regions R3 and R4, revealing that it is suggested that an increased level of vasopressin and/or activation of the renin-angiotensin system may have occurred in addition to excitation of the sympathetic nervous system.

On the correlation diagram at two to three hours after the start of circulation (FIGS. 8c, 9c), no change is seen in ΔBP, ΔSpO₂, and ΔHR both for the extracorporeal circulation group and the target liquid purification group, and data are plotted around the origin.

On the correlation diagram at three to four hours after the start of circulation (FIGS. 8d, 9d), decrease in HR was observed for the extracorporeal circulation group (FIG. 8d), while increase in the blood oxygen saturation as well as decrease in the heart rate were observed for the target liquid purification group (FIG. 9d). For the extracorporeal circulation group, plots are in region R2 and also in regions R3 and R4, suggesting that the sympathetic nervous system was suppressed and vasodilating action in addition to the sympathetic nervous system caused a decrease in the blood oxygen saturation in both groups.

Measuring the blood oxygen saturation and the heart rate together with the blood pressure during extracorporeal circulation in this experiment showed that the arterial blood pressure, the blood oxygen saturation, and heart rate fluctuated with similar trends during extracorporeal circulation and target liquid purification, but the range of fluctuation in the blood oxygen saturation was larger in the target liquid purification. Representing these results on ΔBP-ΔSpO₂-ΔHR correlation diagrams, it was suggested that the factors in blood pressure fluctuation, such as excitation and suppression of the sympathetic nerves, can be visually estimated.

It is known that contact of blood with the target liquid purifying membrane 102 causes a biological incompatibility response such as production of cytokine (Non-Patent Literature 13). A correlation diagram makes it possible to easily identify differences between the extracorporeal circulation group and the target liquid purification group. A correlation diagram also facilitates distinguishing differences between the extracorporeal circulation group and the target liquid purification group. A correlation diagram is thus simple and considered to be useful in understanding the factors of blood pressure fluctuation during dialysis. At present, monitoring of circulation dynamics is performed based on blood pressure and heart rate, or for the autonomic nervous system, on R-R interval (Non-Patent Literature 14). This Example showed that particularly useful information on blood pressure fluctuations during hemodialysis can be obtained by adding a kind of monitoring that reflects vasoconstriction and vasodilation, such as blood oxygen saturation.

The data used in the present invention was acquired from experiments with normal rats. A hemodialysis patient can exhibit continuous hyperactivity of the sympathetic nervous system, which results from a signal generated in a damaged kidney transmitting to the cardiovascular center in the brainstem by way of the afferent renal nerve (Non-Patent Literature 15). A diabetic hemodialysis patient also often develops autonomic disorder due to decrease in blood flow in nerve cells caused by high blood sugar, nerve hypoxia, and decrease in nerve conduction velocity (Non-Patent Literature 16). Hence, when clinical data for hemodialysis patients is compared with the current results with normal rats, varying results will be obtained depending on the conditions of the patients due to difference in the patients' underlying diseases. Therefore, use of the present analysis method is useful for blood pressure management on dialysis patients.

Thus, with the diagnosis assistance image display device, the target liquid purification device or the target liquid purification system of the present invention, change in the mechanism of blood pressure fluctuation can be learned by looking at the heart rate and the blood oxygen saturation, which is an indicator that reflects vasoconstriction, at the same time as the blood pressure fluctuation. It is then possible to manage a target liquid purification process and the like according to the mechanism of blood pressure fluctuation, which in turn enables target liquid purification process to be carried out safely while managing the blood pressure appropriately. Additionally, detrimental effects on patients that may result from medical accidents and the like can be reduced because the condition of a patient receiving target liquid purification, such as a dialysis patient, can be quickly recognized.

While the present invention has been described on the basis of embodiments and examples, the present invention is applicable within a range the present invention is directed to and within a range falling in its technical scope, aside from the embodiments and examples described above. The embodiments and examples of the present invention are also subject to modifications and variations by those skilled in the art within a range of easy modifications and variations.

### Reference Signs List

- A: target liquid purification device
- B: diagnosis assistance image display device
- 1: main unit
- 2: first measurement device
- 3: second measurement device
- 4: third measurement device
- 11: unpurified target liquid path
- 12: purified target liquid path
- 21: filtration and dialysis liquid path
- 22: regenerated liquid path
- 24: waste liquid path
- 100: separator
- 102: target liquid purifying membrane
- 110: first section
- 120: second section
- 200: regenerating section
- 210: replenishing liquid and regenerated liquid concentration device
- 412: first replenishing liquid path
- F11: unpurified target liquid
- F12: purified target liquid
- F21: filtration and dialysis liquid
- F211: first liquid (waste liquid)
- F212: second liquid
- F22: regenerated liquid
- F41: first replenishing liquid

## Claims

1. A diagnosis assistance image display device, the diagnosis assistance image display device displaying, on a coordinate system having a first variable indicating a parameter related to peripheral circulation or temporal fluctuation thereof on a first axis and a second variable indicating a parameter related to blood flow rate or temporal fluctuation thereof on a second axis, a plot that uses variation of a motif to represent variation in a value of a third variable indicating a blood pressure at a predetermined time or temporal fluctuation of the blood pressure over a predetermined period of time, at coordinates of a value of the first variable and a value of the second variable for the predetermined time or the predetermined period of time.

2. The diagnosis assistance image display device according to claim 1, wherein the motif represents time-series fluctuations of at least one of the first variable, the second variable, and the third variable.

3. The diagnosis assistance image display device according to claim 1, wherein a region identifying a factor that affects blood pressure is displayed on the coordinate system, the region enclosed by a line.

4. A target liquid purification device for removing at least one target substance from unpurified target liquid of a patient, the target liquid purification device comprising:
a first measurement device for measuring a first variable indicating a parameter related to peripheral circulation of the patient or temporal fluctuation thereof;
a second measurement device for measuring a second variable indicating a parameter related to blood flow rate of the patient or temporal fluctuation thereof; and
a third measurement device for measuring a third variable indicating a parameter related to blood pressure of the patient or temporal fluctuation thereof.

5. The target liquid purification device according to claim 4, comprising:
a control unit that controls operations of the target liquid purification device based on at least one variable among the first variable measured by the first measurement device, the second variable measured by the second measurement device, and the third variable measured by the third measurement device.

6. A target liquid purification system comprising:
the diagnosis assistance image display device according to claim 1; and
the target liquid purification device according to claim 4.

7. The target liquid purification system according to claim 6, wherein the target liquid purification system performs processing for storing, in a storage device, at least one variable among the first variable measured by the first measurement device, the second variable measured by the second measurement device, and the third variable measured by the third measurement device.
